# EUROPÄISCHE PATENTANMELDUNG

(11) **EP 1 464 409 A2**
(43) Veröffentlichungstag der Anmeldung: **06.10.2004**
(21) Anmeldenummer: 04007744.8
(22) Anmeldetag: 31.03.2004
(51) Int. Cl.: B05B 11/02

(54) **Austragvorrichtung für zumindest ein Medium**

(30) Priorität: 03.04.2003 DE 10315936
(71) Anmelder: ING. ERICH PFEIFFER GMBH, 78315 Radolfzell (DE)
(72) Erfinder: Fuchs, Karl-Heinz, 78315 Radolfzell (DE)
(74) Vertreter: Patentanwälte Ruff, Wilhelm, Beier, Dauster & Partner

(57) **Zusammenfassung**

Austragvorrichtungen für zumindest ein Medium, mit einem Medienspeicher, einer Pumpeinrichtung und einer Austragöffnung sind bekannt.

Erfindungsgemäß weist der Medienspeicher zumindest zwei starr zueinander positionierte Medienspeicherabschnitte (4,6) auf, die in ihrem Inneren durch einen Stufenabsatz ineinander übergehen.

## Beschreibung

Die Erfindung betrifft eine Austragvorrichtung für zumindest ein Medium, mit einem Medienspeicher, einer Pumpeinrichtung und einer Austragöffnung.

Derartige Austragvorrichtungen sind aus dem Stand der Technik in vielfältigen Ausführungen bekannt. Ein Austrag eines oder mehrerer Medien, insbesondere pulverisierter Feststoffe, Flüssigkeiten mit wässriger bis zähfließender Konsistenz oder Gasen, ist insbesondere für viele Bereiche der Kosmetik und Pharmazie von großer Bedeutung. Dabei wird das zumindest eine Medium in einem Medienspeicher der Austragvorrichtung gelagert und kann mittels einer Pumpeinrichtung aus dem Medienspeicher durch eine Austragöffnung hindurch in die Umgebung befördert werden. Die Austragöffnung ist dabei auf die Viskosität und den Anwendungsbereich des auszutragenden Mediums abgestimmt. Insbesondere für Medien mit geringer Partikelgröße, wässriger Konsistenz und niedriger Viskosität wird häufig eine Zerstäubung in der Umgebungsluft mittels einer düsenförmigen Austrittsöffnung angestrebt. Für zähflüssige, hochviskose Flüssigkeiten wird insbesondere eine zylindrische oder konische Austragöffnung bevorzugt. Die Pumpeinrichtungen zum Austragen eines Mediums aus dem Medienspeicher können als selbstansaugende Pumpen ausgelegt sein, bei denen der zum Austrag erforderliche Druck außerhalb des Medienspeichers in der Pumpeinrichtung als solcher aufgebaut wird. Andere Pumpeinrichtungen setzen durch entsprechende Mittel den Medienspeicher unter Druck und bewirken damit den Austrag des Mediums durch die Austragöffnung. Bekannte Medienspeicher weisen einen im wesentlichen gleichförmigen Querschnitt auf und sind hinsichtlich ihrer Wandstärke auf die Erfordernisse der Pumpeinrichtung abgestimmt. Austrageinrichtungen, die für eine geringe Anzahl von Pumphüben, insbesondere für ein oder zwei Pumphübe ausgelegt sind, weisen häufig einen versiegelten Medienspeicher auf. Dazu wird die Pumpeinrichtung so ausgelegt, dass das auszutragende Medium erst unmittelbar bei der Durchführung des Pumpenhubes aus dem bis zu diesem Zeitpunkt versiegelten Medienspeicher herausbefördert wird. Die Pumpeinrichtung öffnet beim ersten Pumpenhub die Versiegelung des Medienspeichers und setzt diesen unter Druck. Derartige Austragvorrichtungen, die für eine geringe Anzahl von Pumphüben ausgelegt sind, werden insbesondere für die Verabreichung von Impfstoffen benutzt. Da hierbei insbesondere nur ein ein- oder zweimaliges Betätigen der Pumpeinrichtung notwendig ist und die Austragvorrichtung aus hygienischen Gründen danach zu entsorgen ist, besteht ein großes Interesse an besonders preisgünstigen und dennoch zuverlässigen Austragvorrichtungen. Aus dem Stand der Technik sind dazu verschiedene Lösungsvarianten bekannt, die insbesondere einen Medienspeicher aus Kunststoff aufweisen. Ein Medienspeicher aus Kunststoff zeichnet sich durch besonders geringe Herstellkosten aus, weist aber Nachteile im Bereich der Langzeitstabilität und der Dichtigkeit auf. Dabei beeinflussen sowohl Diffusionsvorgänge als auch die Qualität der Dichtflächen die Lagerungsfähigkeit des gespeicherten Mediums. Diese Nachteile treten noch stärker bei Medienspeichern zutage, die zwei unterschiedliche Medien, insbesondere ein flüssiges und ein festes Medium beinhalten. Bei derartigen Medienspeichern sind besonders hohe Anforderungen an den Dichtbereich zwischen dem festen und dem flüssigen Medium zu stellen. Nur so kann eine Degeneration der beiden voneinander getrennten Bestandteile verhindert werden. Bekannte Lösungen können hierfür keine vollständig befriedigende Antwort geben.

Die Aufgabe der Erfindung besteht darin, eine Austragvorrichtung der eingangs genannten Art zu schaffen, die eine verbesserte Mischung mehrerer Medien und gute Dosierbarkeit wenigstens eines Mediums für einen Austrag ermöglicht.

Diese Aufgabe wird dadurch gelöst, dass der Medienspeicher zumindest zwei starr zueinander positionierte Medienspeicherabschnitte aufweist, die in ihrem Inneren durch einen Stufenabsatz ineinander übergehen. Der Medienspeicher ist in zumindest zwei Medienspeicherabschnitte unterteilt, die unterschiedliche Querschnitte aufweisen und durch eine feste Verbindung starr zueinander positioniert sind. Die Querschnittsänderung zwischen den beiden Medienspeicherabschnitten geschieht in Form eines Stufenabsatzes, der den Übergang zwischen den zumindest zwei unterschiedlichen Querschnitten herstellt. Der Stufenabsatz überbrückt mit einer insbesondere ebenen Differenzfläche eine sprunghafte Querschnittsveränderung zwischen den beiden unterschiedlichen Querschnitten der Medienspeicherabschnitte. Dadurch ist insbesondere in Verbindung mit wenigstens einem dem Medienspeicherabschnitt zugeordneten Pumpkolbenabschnitt eine exakte Dosierung sowie bei wenigstens zwei Medien eine besonders gute Mischbarkeit dieser Medien erzielbar.

In Ausgestaltung der Erfindung weist der Stufenabsatz eine scharfe, umlaufende Kante auf. Zwischen den Medienspeicherabschnitten wird die sprunghafte Querschnittsveränderung durch den Stufenabsatz überbrückt. An einer Übergangsstelle zwischen dem insbesondere flächigen Stufenabsatz und der Innenfläche des Medienspeicherabschnittes mit dem kleineren Querschnitt durchdringt die Innenfläche des Medienspeicherabschnitts die Fläche des Stufenabsatzes, wobei die beiden Flächen entlang einer Schnittkante ineinander übergehen. Diese Schnittkante ist je nach Ausgestaltung der beiden vorab beschriebenen Flächen in einer Ebene oder im Raum angeordnet, insbesondere bei einer zylindrischen Innenfläche und einem ebenen Stufenabsatz ergibt sich eine ebene, kreisförmige Schnittkante, wobei die beiden Flächen insbesondere in einem Winkel von 90° zueinander liegen. Eine scharfe Kante zwischen den beiden Flächen kann insbesondere in einem Winkelbereich von 20° bis 135°, in bevorzugter Ausführung von 80° bis 110°, verwirklicht werden. Durch die scharfe Kante wird ein besonders guter Sitz eines in diesem Bereich angeordneten Dichtmittels gewährleistet. Weiterhin ergibt sich durch die scharfe Kante, insbesondere bei einer Verwendung des Medienspeichers zur Lagerung von zumindest zwei unterschiedlichen Medien durch die strömungstechnischen Eigenschaften einer derartigen Kante eine besonders vorteilhafte Durchmischung der entsprechenden Medien. Bei lediglich einem Medium ist eine besonders exakte Dosierung erreichbar.

In weiterer Ausgestaltung der Erfindung sind die Medienspeicherabschnitte durch zwei getrennte Hohlkörper gebildet, die einander über einen Teilbereich ihrer Länge überlagern und in dem Überlagerungsbereich dicht miteinander verbunden sind. Durch eine Ausführung der Medienspeicherabschnitte als getrennte Hohlkörper können für die zu speichernden Medien unterschiedliche Werkstoffe für die Medienspeicherabschnitte eingesetzt werden. Die Werkstoffe können somit insbesondere auf die Bedürfnisse des jeweiligen Mediums speziell abgestimmt werden. Zur Schaffung eines einheitlichen Medienspeichers werden die entsprechenden Hohlkörper über einen Teilbereich ihrer Länge miteinander überlagert und in dem dadurch gebildeten Überlagerungsbereich dicht miteinander verbunden.

In weiterer Ausgestaltung der Erfindung ist zumindest ein Medienspeicherabschnitt aus einem kristallinen oder amorphen Werkstoff hergestellt und weist eine nahezu glatte Innenwand auf. Kristalline und amorphe Werkstoffe, insbesondere Metalle, Keramiken oder Gläser, sind für die Speicherung von Medien über einen längeren Zeitraum besonders gut geeignet. Sie lassen durch ihre hohe Dichte nur eine extrem geringe Diffusion zwischen dem zu speichernden Medium, dem Werkstoff des Medienspeichers und der Umgebung zu. Darüber hinaus verhalten sich derartige Werkstoffe inert zu den zu speichernden Medien. Auch bei langer Lagerungszeit des gefüllten Medienspeichers besteht nur eine geringe Gefahr der Wechselwirkung des Mediums mit dem Werkstoff des Medienspeichers. Ein weiterer Vorteil derartiger Werkstoffe liegt darin, dass sie mittels großserientauglicher und kostengünstiger Fertigungsverfahren mit nahezu glatten Innenwänden hergestellt werden können. Dadurch wird einerseits die wirksame Fläche für einen Austausch zwischen Medium und dem Werkstoff des Medienspeicherabschnitts deutlich reduziert. Weiterhin kann dadurch auch ein besonders vorteilhafter Sitz der Dichtungselemente bewirkt werden. Ein weiterer Vorzug einer glatten Innenwand tritt während des Austragvorganges zutage, insbesondere wenn zumindest ein Medienspeicherabschnitt auch als Pumpenzylinder genutzt wird. Hierbei trägt eine glatte Innenwand zu einer besonders reibungsarmen und effizienten Pumpbewegung des insbesondere als Dichtungselement ausgeführten Pumpenkolbens bei.

In weiterer Ausgestaltung der Erfindung ist eine Außenkontur des inneren Hohlkörpers zumindest abschnittsweise auf eine Innenkontur des äußeren Hohlkörpers abgestimmt. Durch die Abstimmung der Außenkontur des inneren Hohlkörpers auf die Innenkontur des äußeren Hohlkörpers, insbesondere bei Verwendung von ISO-Toleranzfeldern, lässt sich eine sichere und vorteilhafte Montage sowie eine zuverlässige Dichtwirkung zwischen den beiden Medienspeicherabschnitten erzielen.

Darüber hinaus wird, insbesondere bei der Verwendung eines amorphen Werkstoffes wie Glas vermieden, dass bei der Montage der Medienspeicherabschnitte innere Spannungen auftreten, die zu einer Schädigung der Medienspeicherabschnitte oder zu einer Beeinträchtigung der Dichtigkeit des Überlagerungsbereiches führen könnten.

In weiterer Ausgestaltung der Erfindung sind die zumindest zwei Hohlkörper in dem Überlagerungsbereich stoffschlüssig verbunden. Der Stoffschluss im Überlagerungsbereich kann insbesondere durch Verschmelzen, Verlöten oder Verkleben der Werkstoffe der Medienspeicherabschnitte verwirklicht werden. Das Verschmelzen der Werkstoffe der Medienspeicherabschnitte erfolgt insbesondere durch Zufuhr thermischer Energie mittels offener Flamme, elektrischem Lichtbogen, Reibungswärme, Laserlicht oder Induktion. Bei Lötverfahren wird ein Zusatzwerkstoff mit einem Schmelzpunkt unterhalb des oder der Schmelzpunkte der zu verbindenden Werkstoffe eingesetzt, der in einen Kapillarspalt des Überlagerungsbereiches durch Erwärmung der Medienspeicherabschnitte eingebracht werden kann. Zur Verklebung der Medienspeicherabschnitte werden insbesondere ein- oder zweikomponentige Klebstoffe eingesetzt, die anaerob, durch externe Energiezufuhr, durch chemische Reaktion mit einem Härter, durch Luft- oder Feuchtigkeitskontakt oder mittels weiterer Mechanismen aktiviert und ausgehärtet werden.

In weiterer Ausgestaltung der Erfindung ist zumindest der Überlagerungsbereich zumindest eines Hohlkörpers zylindrisch geformt und weist insbesondere eine Schäftung auf. Die zylindrische Gestaltung des Überlagerungsbereichs zumindest eines Hohlkörpers stellt eine besonders kostengünstig herzustellende Geometrie für einen Medienspeicherabschnitt dar. Weitere Vorteile liegen in der Verbindung mit dem angrenzenden Medienspeicherabschnitt. Eine zylindrische Gestaltung vermeidet Spannungsspitzen in den zu verbindenden Medienspeicherabschnitten und reduziert somit das Risiko von Rissbildung und Undichtigkeit. Eine Schäftung des zylindrisch geformten Überlagerungsbereichs des Medienspeicherabschnittes wird insbesondere durch eine konische Gestaltung des Überlagerungsbereiches erreicht. Der Konus weist an der dem anderen Medienspeicherabschnitt zugewandten Stirnseite einen geringeren Durchmesser auf, mit zunehmendem Abstand von dem anderen Medienspeicherabschnitt ist eine Durchmesserzunahme vorgesehen.

In weiterer Ausgestaltung der Erfindung weist jeder Medienspeicherabschnitt eine von dem benachbarten Medienspeicherabschnitt getrennte Kammer für jeweils ein Medium auf. Somit können in dem Medienspeicher zwei völlig unterschiedliche Medien, insbesondere in flüssigem, festem oder gasförmigem Aggregatszustand aufbewahrt werden, die erst unmittelbar zum Zeitpunkt des Austrages miteinander vermischt werden. Dazu wird der Medienspeicher auf Höhe des Stufenabsatzes mittels eines Dichtelementes in zwei Kammern aufgetrennt. Insbesondere durch die dem Stufenabsatz zugeordnete, scharfe, umlaufende Kante sowie die nahezu glatte Innenwand zumindest eines Medienspeicherabschnittes wird hierbei eine besonders vorteilhafte Dichtwirkung erzielt. Bei einem Austragvorgang wird dieses Dichtelement aus einer Dichtposition insbesondere in eine der Kammern des Medienspeichers verschoben, dadurch tritt insbesondere die gewünschte Durchmischung der zumindest zwei unterschiedlichen Medien im Medienspeicher ein. Nachfolgend kann das Mediengemisch durch die Austragöffnung in die Umgebung abgegeben werden. Durch die scharfe Kante des Stufenabsatzes wird insbesondere die Mischwirkung unterstützt und kann dadurch in einem kurzen Zeitabschnitt gewährleistet werden. Je nach Anwendungsfall können sowohl flüssige als auch feste Medien in den Medienspeicherabschnitten vorgesehen werden, die je nach Mischungsverhältnis in den volumenmäßig entsprechend angepassten Medienspeicherabschnitten eingebracht werden.

In weiterer Ausgestaltung der Erfindung ist in zumindest einem Dichtbereich an einer der Medienkammer zugewandten Innenwandung eines Medienspeicherabschnittes eine umlaufende Ausformung zur Aufnahme eines zumindest abschnittweise kugelförmigen Dichtelementes vorgesehen ist. Derartige Dichtelemente werden insbesondere aus Elastomerwerkstoffen hergestellt und bieten insbesondere bei der Dichtungsmontage Vorteile gegenüber flächigen Dichtelementen mit zylindrischem Querschnitt. Dies resultiert insbesondere aus der Kugelform, die einerseits ein leichtes Einbringen des Dichtelementes in die im Dichtbereich vorgesehene Ausformung ermöglicht. Weiterhin ist ein zumindest abschnittsweise kugelförmiges Dichtelement nahezu indifferent gegenüber einer Ausrichtung relativ zum Dichtbereich. Die umlaufende Ausformung im Dichtbereich an der Innenwandung des Medienspeicherabschnittes kann insbesondere als Nut- oder als Vorsprungsanordnung ausgeführt sein. Dadurch greift das zumindest abschnittsweise kugelförmige Dichtelement zumindest abschnittsweise formschlüssig in den Dichtbereich ein.

In weiterer Ausgestaltung der Erfindung sind als Hohlkörper konzentrisch angeordnete, zylindrische Glasrohrabschnitte vorgesehen, die in dem Überlagerungsbereich insbesondere miteinander laserverschweißt sind. Glasrohrabschnitte lassen sich industriell mit extrem genauen Querschnitten besonders kostengünstig herstellen und können in nahezu beliebiger Menge bezogen werden. Eine individuelle Anpassung der zylindrischen Glasrohrabschnitte im Überlagerungsbereich bei der Montage ist in Anbetracht der hohen Maßhaltigkeit und der geringen Toleranzen derartiger Hohlkörper nicht erforderlich. Dadurch kann eine besonders gute Überlagerung der Medienspeicherabschnitte bei extrem geringen Zusatzkosten verwirklicht werden. Durch die Ausführung des Medienspeichers aus zylindrischen Glasrohrabschnitten lässt sich auch langfristig eine extrem gute Dichtwirkung sowohl im Hinblick auf Dichtungselemente im Überlagerungsbereich als auch hinsichtlich der stirnseitig an den Hohlkörperenden angeordneten Dichtungselemente verwirklichen. Die stoffschlüssige Verbindung zwischen den Medienspeicherabschnitten kann insbesondere durch Laserverschweißen mit hoher Prozesssicherheit gasdicht hergestellt werden. Die Herstellung dieser Verbindung bleibt gleichzeitig ohne Auswirkungen auf eine Maßhaltigkeit des Medienspeichers, da bei Verwendung eines Laserschweißprozesses lediglich die äußeren Randzonen der miteinander zu verbindenden Medienspeicherabschnitte aufgeschmolzen werden. Die Innenbereiche der zylindrischen Glasrohrabschnitte erfahren keine Aufschmelzung und somit auch keine Deformation. Vorteilhaft lässt sich diese Ausgestaltung mit dem Merkmal einer Schäftung kombinieren.

In weiterer Ausgestaltung der Erfindung ist an der Pumpeinrichtung zumindest eine kraftbegrenzte Rückhalteeinrichtung vorgesehen, die einen Pumpenhub unterhalb eines definierten Kraftniveaus verhindert. Um eine ungewollte Betätigung sowie einen unzureichenden Druckaufbau im Medienspeicher zu verhindern, ist an der Pumpeinrichtung eine kraftbegrenzte Rückhalteeinrichtung vorgesehen. Für einen Pumpenhub muss der Benutzer die Austragvorrichtung insbesondere mittels Fingerkraft unter Druck setzen. Erst bei Überschreiten eines vordefinierten Kraftniveaus wird der Pumpenhub von der Rückhalteeinrichtung freigegeben, wobei durch die Trägheit eines aus der Austragvorrichtung und den Fingern des Bedieners gebildeten Systems eine definierte Geschwindigkeit des Pumpenhubs nicht mehr unterschritten werden kann. Kraftbegrenzte Rückhaltevorrichtungen können insbesondere in Form von Rastkanten oder Abreißelementen verwirklicht werden.

In weiterer Ausgestaltung der Erfindung sind kraftbegrenzte Rückhalteeinrichtungen mit unterschiedlichen Sperrkräften für eine definierte Abfolge einzelner Schritte des Pumpenhubes vorgesehen. Durch derartige kraftbegrenzte Rückhalteeinrichtungen kann bewirkt werden, dass eine vorgesehene Bedienabfolge in der Austragvorrichtung exakt eingehalten wird und somit die insbesondere bei Medienspeichern mit zwei oder mehreren getrennten Kammern notwendige Durchmischung der unterschiedlichen Medien bestimmungsgemäß erfolgt. Die unterschiedlichen Sperrkräfte werden durch unterschiedliche Auslegung der Rückhalteeinrichtungen verwirklicht, insbesondere kann durch Kombination einer Rastkante mit einem Abreißelement eine zuverlässige Differenzierung unterschiedlicher Sperrkräfte erreicht werden. Dadurch kann ein gezielt gesteuerter Ablauf des Pumpenhubes erreicht werden. Insbesondere wird die Sperrkraft der als Rastkante vorgesehenen Rückhalteeinrichtung zuerst überwunden und dadurch ein oder mehrere Schritte einer ersten Phase des Pumpenhubes absolviert. Erst anschließend wird die Sperrkraft der als Abreißelement verwirklichten Rückhalteeinrichtung überschritten und eine zweite Phase des Austraghubes vollzogen.

Weitere Vorteile und Merkmale der Erfindung ergeben sich aus den Ansprüchen und der nachfolgenden Beschreibung einer bevorzugten Ausführungsform der Erfindung, die anhand der Zeichnungen dargestellt sind.
- Fig.1: zeigt in ebener Ansicht eine schematische Schnittdarstellung einer Austragvorrichtung mit einem Medienspeicher,
- Fig.2: in ebener Ansicht eine Schnittdarstellung einer zweiten Ausführungsform einer Austragvorrichtung mit einem Medienspeicher
- Fig.3: in ebener Ansicht eine Schnittdarstellung eines Medienspeichers der zweiten Ausführungsform der Austragvorrichtung.

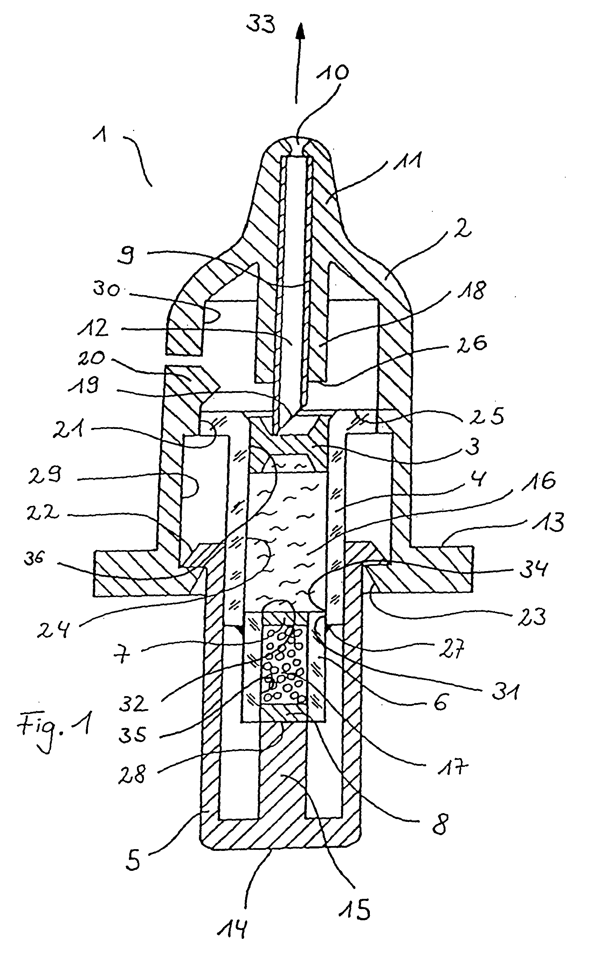

Eine in Fig. 1 in schematischer Ausführung dargestellte Austragvorrichtung 1 weist einen Grundkörper 2 auf, der alle weiteren Bestandteile der Austragvorrichtung beinhaltet oder zumindest abschnittsweise umfasst.

Der Grundkörper 2 weist an einem in Austragrichtung 33 gelegenen Ende einen Düsenschaft 11 sowie eine als Düse 10 ausgeformte Austragöffnung auf. Der Düsenschaft 11 ist bei dem vorliegenden Ausführungsbeispiel insbesondere als Nasenolive ausgeführt und weist eine im wesentlichen verrundete, kegelabschnittsförmige Kontur auf. An einem einer Austragrichtung 33 abgewandten Ende ist der Grundkörper 2 mit einer zumindest abschnittsweise umlaufenden Fingerauflage 13 ausgestattet. Die Fingerauflage 13 ist zum Hervorrufen einer Relativbewegung zwischen dem Grundkörper 2 und einer Druckhülse 5, insbesondere als Haltefläche für einen Zeigefinger und einen Mittelfinger eines Benutzers, vorgesehen. Der Daumen des Benutzers wird zur Erzeugung der für einen Pumpenhub notwendigen Kraft auf einer Grundfläche 14 der Druckhülse 5 abgelegt. Somit wird ein Kraftfluss zwischen der Fingerauflage 13, dem Grundkörper 2, den weiteren Bestandteilen der Austragvorrichtung 1 sowie der Hand des Benutzers ermöglicht. Durch die Relativbewegung zwischen dem Grundkörper 2 und der Druckhülse 5 wird der Pumpenhub und somit der Austrag des Mediums oder der Medien bewirkt.

Die Druckhülse 5 ist mittels einem Außenrastkonus 22, der bei der Montage der Austragvorrichtung durch einen flexiblen Innenrastkonus 23 des Grundkörpers 2 hindurchgeschoben wird, im Inneren des Grundkörpers formschlüssig verrastet. Der Formschluss der Druckhülse 5 verhindert ausschließlich eine Demontage aus dem Grundkörper 2. Eine Beweglichkeit der Druckhülse 5 in Richtung der Austragrichtung 33 wird durch den Formschluss zwischen Druckhülse 5 und Grundkörper 2 nicht eingeschränkt. Der Außenrastkonus 22 der Druckhülse 5 wird während der Austragbewegung entlang einer Führungsbuchse 29 des Grundkörpers 2 geführt und ermöglicht somit eine gerade Bewegung der Druckhülse 5 relativ zum Grundkörper 2. Die Druckhülse 5 weist einen im wesentlichen becherartigen Querschnitt auf, wobei am Boden des becherartigen Querschnittes ein zentrisch angeordneter und aus der Bodenfläche heraustretender Feststoffstößel 15 vorgesehen ist. Der Feststoffstößel liegt in einer Ruheposition der Druckhülse 5 unmittelbar an einer Außendichtung 8 des Feststoffspeichers 6 an.

Der Feststoffspeicher 6 ist ein Medienspeicherabschnitt, der aus einem zylindrischen Glasrohrabschnitt hergestellt ist, und der mittels eines Überlagerungsbereiches 31 sowie einem als Laserschweißnaht ausgeführten Verbindungsbereich 27 mit einem weiteren Medienspeicherabschnitt in Form eines Flüssigkeitsspeichers 4 gasdicht verbunden ist. Der im Feststoffspeicher 6 gelagerte Feststoff 17 wird durch eine Innendichtung 7, die an einer in Austragrichtung von der Außendichtung 8 beabstandeten Stirnfläche des Feststoffspeichers 6 vorgesehen ist, von einem in dem Flüssigkeitsspeicher 4 enthaltenen Flüssigmedium 16 abgetrennt. Die Innendichtung 7 stützt sich an einer als Dichtfläche wirkenden Zylinderfläche 35 des Feststoffspeichers 6 ab. An der Stirnfläche des Feststoffspeichers 6 ist auch die Medienspeicherkante 32 angeordnet, die sich durch den Querschnittsunterschied des Flüssigkeitsspeichers 4 zum Feststoffspeicher 6 ergibt und die an einem inneren Bereich des Stufenabsatzes 34 angeordnet ist. Der Feststoffspeicher 6 ist in den Flüssigkeitsspeicher 4 geschäftet, was aus der Zeichnung aber nicht erkennbar ist.

Im Flüssigkeitsspeicher 4 ist ein Flüssigmedium 16 gelagert, das an einer der Austragöffnung 10 zugewandten Stirnseite durch eine Kolbendichtung 3 abgedichtet wird. Die Kolbendichtung stützt sich an einer Zylinderfläche 24 des Flüssigkeitsspeichers 4 ab und ist bedingt durch ihre Geometrie und die geringe Rauhigkeit der Zylinderfläche 24 während des Austragvorganges leicht verschieblich. Unmittelbar über der Kolbendichtung 3 ist in den Grundkörper 2 das Steigrohr 9 eingelassen, das in einem dem Medienspeicher zugewandten Abschnitt des Grundkörpers 2 durch einen Flüssigkeitsstößel 18 abgestützt wird und das an seiner dem Medienspeicher zugewandten Stirnseite mit einer Steigrohrschneide 19 versehen ist.

Bevor ein Pumpenhub ausgeführt werden kann, muss der Benutzer durch Aufbringen einer Kraft zwischen der Fingerauflage 13 und der Druckfläche 14 den durch die als Rastkante 20 ausgeführte kraftbegrenzte Rückhalteeinrichtung definierten Mindestdruck ausüben. Erst nach Überwindung der Rastkante 20 wird eine Austragbewegung der Druckhülse 5 sowie des Medienspeichers relativ zum Grundgehäuse möglich. Zu Beginn eines Austraghubes wird durch die Steigrohrschneide 19 die Kolbendichtung 3 in einem verjüngten Bereich durchtrennt. Sobald das Steigrohr 9 die Kolbendichtung 3 vollständig durchdrungen hat und von dieser umlaufend umfasst wird, kommt der Flüssigkeitsstößel 18 mit seiner Kolbendruckfläche 26 in Berührung mit der Kolbendichtung 3 und setzt mittels der durch den Bediener ausgeübten Kraft den Medienspeicher unter Druck. Durch die vom Bediener ausgeübte Kraft wird zeitgleich auch der Feststoffspeicher 6 mittels der auf die Druckfläche 14 über den Feststoffstößel 15 auf die Außendichtung 8 übertragenen Kraft ebenfalls unter Druck gesetzt. Dadurch wird die Innendichtung 7 in den Flüssigkeitsspeicher 4 geschoben und eine Durchmengung des Feststoffes 17 und des Flüssigmediums 16 bewirkt. In dem vorliegenden Ausführungsbeispiel ist die Austragvorrichtung 1 als Einmaldosiervorrichtung ausgeführt, d.h. die in den Kammern des Medienspeichers gelagerten Medien 16, 17 werden mittels eines einzigen Pumpenhubs durchmischt und durch den Medienkanal 12 des Steigrohres 9 in Austragrichtung 33 durch die Düse 10 der Austragvorrichtung befördert.

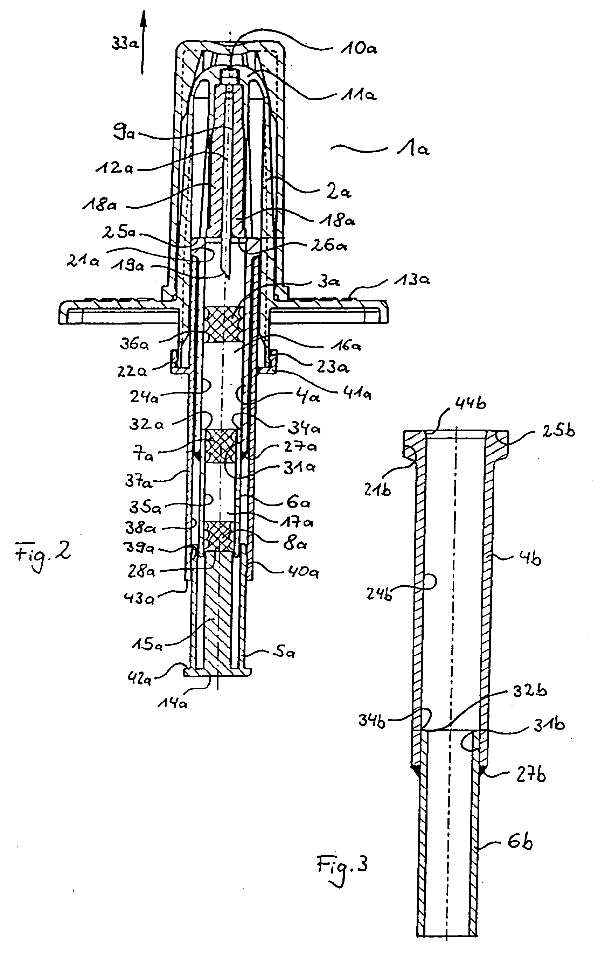

Abweichend von der in Fig. 1 dargestellten schematischen Austragvorrichtung ist in Fig.2 eine tatsächliche Ausführungsform der Austragvorrichtung 1a dargestellt. In der folgenden Beschreibung werden lediglich die Abweichungen von der Darstellung aus Fig.1 geschildert. Bei der in Fig. 2 dargestellten Austragvorrichtung 1a ist zwischen dem Grundkörper 2a und der Druckhülse 5a eine zusätzliche Zwischenhülse 37a vorgesehen. Die Zwischenhülse 37a weist an einem der Druckhülse 5a zugewandten Ende an einer Innenführungsfläche 38a eine Rastausnehmung 39a auf , die zusammen mit einem Rastbund 40a der Druckhülse 5a eine erste, formschlüssig wirkende kraftbegrenzte Rückhalteeinrichtung bildet. An einer dem Grundkörper 2a zugewandten Seite ist die Zwischenhülse 37a mit einem umlaufenden Bundsteg 41a versehen, der einen Innenrastkonus 23a aufweist. Dieser Innenrastkonus 23a bildet mit dem Außenrastkonus 22a eine formschlüssige Verbindung, die bei der Montage der Austragvorrichtung 1a hergestellt wird und der Halterung des Medienspeichers im Grundkörper 2a dient. Dadurch wird weiterhin eine zweite kraftbegrenzte Rückhalteeinrichtung gebildet. Die Begrenzung der Betätigungskraft dieser zweiten kraftbegrenzten Rückhalteeinrichtung wird durch den als Abreißring ausgeführten Bundsteg 41a verwirklicht.

Zu Durchführung eines Pumpenhubes wird bei der in Fig. 2 dargestellten Austragvorrichtung 1a ebenso wie bei der aus Fig. 1 bekannten Austragvorrichtung 1 eine Kraft zwischen der Fingerauflage 13a und der Druckfläche 14a der Druckhülse 5a auf die Austragvorrichtung 1a ausgeübt. Sobald der Benutzer eine ausreichende Druckkraft auf die Druckfläche 14a aufbringt, kann eine durch die Geometrie der Rastausnehmung 39a sowie des Rastbundes 40a und der entsprechenden Werkstoffeigenschaften bestimmte Sperrkraft dieser kraftbegrenzten Rückhalteeinrichtung überwunden werden. Dadurch setzt sich die Druckhülse 5a in Austragrichtung 33a in Bewegung, wobei sie längs der Innenführungsfläche 38a in der Zwischenhülse 37a gleitet. Durch den an der Druckhülse 5a vorgesehenen Feststoffstößel 15a wird die Außendichtung 8a in Richtung der Austragrichtung 33a bewegt. Dadurch kommt es im Feststoffspeicher 6a zu einem Druckaufbau im Feststoff 17a. Dieser aufgebaute Druck pflanzt sich durch den Feststoff 17a, die Innendichtung 7a und das Flüssigmedium 16a bis zur Kolbendichtung 3a fort. Die Kolbendichtung 3a wird lediglich durch Haftreibungskräfte ortsfest gehalten, sie kann durch Überwindung dieser Haftreibungskräfte in Austragrichtung 33a verschoben werden. Damit wird zeitgleich eine gleichsinnige Bewegung der Außendichtung 8a, des Feststoffs 17a, der Innendichtung 7a und des Flüssigmediums 16a ermöglicht. Sobald die Innendichtung 7a mit ihrem Dichtbereich die Medienspeicherkante 32a passiert hat, tritt eine Durchmischung des Feststoffes 17a mit dem Flüssigmedium 16a ein. Durch die Verschiebung der Kolbendichtung 3a in Austragrichtung 33a wird diese von der Steigrohrschneide 19a durchdrungen. Kurz bevor das Steigrohr 9a die Kolbendichtung 3a vollständig durchdrungen hat, trifft ein Anschlagbund 42a der Druckhülse 5a formschlüssig auf eine der Druckhülse 5a zugewandte Stirnseite 43a der Zwischenhülse 37a auf. Damit erfolgt vorerst kein weiterer Druckaufbau in dem Gemisch aus Flüssigmedium 16a und Feststoff 17a und das Steigrohr 9a ist noch nicht in Kontakt mit dem Gemisch im Medienspeicher. Erst bei Überwindung einer Mindestbetätigungskraft, die zu einem Abreißen des Bundstegs 41a von der Zwischenhülse 37a führt, wird die Kolbendichtung 3a vom Steigrohr 9a vollständig durchdrungen und das zwischenzeitlich entstandene Gemisch aus Flüssigmedium 16a und Feststoff 17a kann schlagartig durch den Medienkanal 12a in die Umgebung entweichen. Dabei wirkt der Flüssigkeitsstößel 18a mit seiner Kolbendruckfläche 26a auf eine der Düse 10a zugewandte Stirnseite der Kolbendichtung 3a und setzt somit das im wesentlichen im Flüssigkeitsspeicher 4a befindliche Gemisch unter Druck, bis eine Stirnanschlagfläche 25a des Flüssigkeitsspeichers 4a auf einen nicht näher bezeichneten Anschlag im Grundkörper aufläuft. Damit ist der Pumpenhub beendet und das Gemisch aus Flüssigmedium 16a und Feststoff 17a bis auf eine Restmenge ausgetragen.

Der in Fig. 3 dargestellte, nicht näher bezeichnete Medienspeicher ist aus einem zylindrischen Glasrohr mit einem stirnseitig angeordneten, nach außen weisenden umlaufenden Bund versehen, der an einer konzentrisch zu einer Zylindermittelachse vorgesehenen Führungsfläche 21b einen exakt bestimmten Außendurchmesser aufweist. Weiterhin ist an einer nach innen gewandten Zylinderfläche 24b des Flüssigkeitsspeichers 4b ein Einführkonus 44b vorgesehen, der sich von einer Stirnanschlagfläche 25b des Flüssigkeitsspeichers 4b auf einen nicht näher bezeichneten Innendurchmesser des Flüssigkeitsspeichers 4b verengt. An einer dem umlaufenden Bund abgewandten Stirnseite des Flüssigkeitsspeichers 4b ist ein abschnittsweise exakt bestimmter Innendurchmesser vorgesehen, der zur Aufnahme eines als zylindrisches Glasrohr ausgeführten Feststoffspeichers 6b vorgesehen ist. Dabei bildet der Feststoffspeicher 6b, der zumindest abschnittsweise einen exakt auf den Innendurchmesser des Flüssigkeitsspeichers 4b abgestimmten Außendurchmesser aufweist, zusammen mit dem Flüssigkeitsspeicher 4b einen Überlagerungsbereich 31b. An einer durch den Durchmesserunterschied des Flüssigkeitsspeichers 4b und des Feststoffspeichers 6b gebildeten, nicht näher bezeichneten Stufe ist ein Verbindungsbereich 27b vorgesehen, der insbesondere durch Laserverschweißen der beiden zylindrischen Glaskörper entsteht. An einer Stirnseite des Feststoffspeichers 6b, die dem Flüssigkeitsspeicher 7b zugewandt ist, ist ein scharfkantiger Stufenabsatz 34b vorgesehen, der sich seinerseits ebenfalls aus den Durchmesserunterschied des Flüssigkeitsspeichers 4b und des Feststoffspeichers 6b ergibt.

Andere Ausgestaltungen der erfindungsgemäßen Austragsvorrichtung können insbesondere als Zweifach- oder Mehrfachdosiersystem ausgeführt sein. Die erfindungswesentlichen Funktionsbereiche ändern sich hierdurch nicht.

### Bezugszeichenliste:

- 1.: Austragvorrichtung
- 2.: Grundkörper
- 3.: Kolbendichtung
- 4.: Flüssigkeitsspeicher
- 5.: Druckhülse
- 6.: Feststoffspeicher
- 7.: Innendichtung
- 8.: Außendichtung
- 9.: Steigrohr
- 10.: Düse
- 11.: Düsenschaft
- 12.: Medienkanal
- 13.: Fingerauflage
- 14.: Druckfläche
- 15.: Feststoffstößel
- 16.: Flüssigmedium
- 17.: Feststoff
- 18.: Flüssigkeitsstößel
- 19.: Steigrohrschneide
- 20.: Rastkante
- 21.: Führungsfläche
- 22.: Außenrastkonus
- 23.: Innenrastkonus
- 24.: Zylinderfläche Flüssigkeitsspeicher
- 25.: Stirnanschlagfläche
- 26.: Kolbendruckfläche
- 27.: Verbindungsbereich
- 28.: Feststoffstößeldruckfläche
- 29.: Führungsbuchse
- 30.: Führungshülse
- 31.: Überlagerungsbereich
- 32.: Medienspeicherkante
- 33.: Austragrichtung
- 34.: Stufenabsatz
- 35.: Zylinderfläche Feststoffspeicher
- 36.: Dichtbereich
- 37.: Zwischenhülse
- 38.: Innenführungsfläche
- 39.: Rastausnehmung
- 40.: Rastbund
- 41.: Bundsteg
- 42.: Anschlagbund
- 43.: Stirnseite
- 44.: Einführungskonus

## Patentansprüche

1. Austragvorrichtung (1, 1a) für zumindest ein Medium (16, 16a, 17, 17a), mit einem Medienspeicher, einer Pumpeinrichtung und einer Austragöffnung (10, 10a), **dadurch gekennzeichnet, dass** der Medienspeicher zumindest zwei starr zueinander positionierte Medienspeicherabschnitte (4, 4a, 4b, 6, 6a, 6b) aufweist, die in ihrem Inneren durch einen Stufenabsatz (34, 34a, 34b) ineinander übergehen.

2. Austragvorrichtung nach Anspruch 1, **dadurch gekennzeichnet, dass** der Stufenabsatz (34, 34a, 34b) eine scharfe, umlaufende Kante (32, 32a, 32b) aufweist.

3. Austragvorrichtung nach Anspruch 1, **dadurch gekennzeichnet, dass** die Medienspeicherabschnitte (4, 4a, 4b, 6, 6a, 6b) durch zwei getrennte Hohlkörper gebildet sind, die einander über einen Teilbereich ihrer Länge überlagern und in dem Überlagerungsbereich (31, 31a, 31b) dicht miteinander verbunden sind.

4. Austragvorrichtung nach Anspruch 1, **dadurch gekennzeichnet, dass** zumindest ein Medienspeicherabschnitt (4, 4a, 4b, 6, 6a, 6b) aus einem kristallinen oder amorphen Werkstoff hergestellt ist und eine nahezu glatte Innenwand (24, 24a, 24b, 35, 35a, 35b) aufweist.

5. Austragvorrichtung nach Anspruch 3, **dadurch gekennzeichnet, dass** eine Außenkontur des inneren Hohlkörpers (6, 6a, 6b) zumindest abschnittsweise auf eine Innenkontur des äußeren Hohlkörpers (4, 4a, 4b) abgestimmt ist.

6. Austragvorrichtung nach Anspruch 3, **dadurch gekennzeichnet, dass** die zumindest zwei Hohlkörper (4, 4a, 4b, 6, 6a, 6b) in dem überlagerungsbereich (31, 31a, 31b) stoffschlüssig verbunden sind.

7. Austragvorrichtung nach Anspruch 5, **dadurch gekennzeichnet, dass** zumindest der Überlagerungsbereich (31, 31a, 31b) zumindest eines Hohlkörpers (4, 4a, 4b, 6, 6a, 6b) zylindrisch geformt ist und insbesondere eine Schaffung aufweist.

8. Austragvorrichtung nach Anspruch 1, **dadurch gekennzeichnet, dass** jeder Medienspeicherabschnitt (4, 4a, 4b, 6, 6a, 6b) eine von dem benachbarten Medienspeicherabschnitt (4, 4a, 4b, 6, 6a, 6b) getrennte Kammer für jeweils ein Medium (16, 16a, 17, 17a) aufweist.

9. Austragvorrichtung nach Anspruch 1, **dadurch gekennzeichnet, dass** in zumindest einem Dichtbereich (36, 36a) an einer der Medienkammer zugewandten Innenwandung (24, 24a, 24b) eines Medienspeicherabschnittes (4, 4a, 4b, 6, 6a, 6b) eine umlaufende Ausformung zur Aufnahme eines zumindest abschnittsweise kugelförmigen Dichtelements (3, 3a, 7, 7a, 8, 8a) vorgesehen ist.

10. Austragvorrichtung nach Anspruch 1, **dadurch gekennzeichnet, dass** als Hohlkörper (4, 4a, 4b, 6, 6a, 6b) konzentrisch angeordnete, zylindrische Glasrohrabschnitte vorgesehen sind, die in dem Überlagerungsbereich (31, 31a, 31b) insbesondere miteinander laserverschweißt sind.

11. Austragvorrichtung nach Anspruch 1, **dadurch gekennzeichnet, dass** an der Pumpeinrichtung zumindest eine kraftbegrenzte Rückhalteeinrichtung (20, 39a, 40a, 41a) vorgesehen ist, die einen Pumpenhub unterhalb eines definierten Kraftniveaus verhindert.

12. Austragvorrichtung nach Anspruch 11, **dadurch gekennzeichnet, dass** kraftbegrenzte Rückhalteeinrichtungen (20, 39a, 40a, 41a) mit unterschiedlichen Sperrkräften für eine definierte Abfolge einzelner Schritte des Pumpenhubes vorgesehen sind.
